# FASCICULE DE BREVET EUROPEEN

(11) **EP 3 407 831 B1**
(45) Date de publication et mention de la délivrance du brevet: **14.09.2022**
(21) Numéro de dépôt: 17709126.1
(22) Date de dépôt: 30.01.2017
(51) Int. Cl.: A61D 19/02

(54) **DISPOSITIF POUR LE FRANCHISSEMENT DU COL DE L'UTERUS D'UN ANIMAL DE RENTE, POUR LE TRANSFERT DE MATERIEL OU SUBSTANCE A BUT REPRODUCTIF, THERAPEUTIQUE OU DIAGNOSTIQUE OU PRELEVEMENT DEPUIS L'UTERUS**
VORRICHTUNG ZUM GELANGEN HINTER DEN GEBÄRMUTTERMUND EINES NUTZVIEHS ZUM ÜBERTRAGEN VON MATERIAL ODER EINER SUBSTANZ FÜR FORTPFLANZUNGS-, THERAPEUTISCHE ODER DIAGNOSTISCHE ZWECKE ODER ZUR PROBENAHME AUS DER GEBÄRMUTTER
DEVICE FOR REACHING PAST THE NECK OF THE UTERUS OF A LIVESTOCK ANIMAL FOR THE PURPOSE OF TRANSFERRING MATERIAL OR SUBSTANCE WITH A REPRODUCTIVE, THERAPEUTIC OR DIAGNOSTIC PURPOSE OR FOR COLLECTING SAMPLES FROM THE UTERUS

(30) Priorité: 29.01.2016 FR 1650764
(43) Date de publication de la demande: 05.12.2018
(73) Titulaire: Elexinn, 89400 Migennes (FR)
(72) Inventeur: DECHERF, Agathe, 89400 Migennes (FR); DREVILLON, Pierrick, 89400 Migennes (FR)
(74) Mandataire: Cabinet Chaillot
(86) Numéro de dépôt international: PCT/FR2017/050211
(87) Numéro de publication internationale: WO 2017/129929

(56) Documents cités:
- EP-A1- 0 157 888
- EP-A1- 0 319 394
- WO-A1-2015/185863
- DE-A1- 2 701 998
- FR-A1- 2 212 132
- US-A- 4 474 576
- US-A- 5 259 836
- US-A1- 2012 323 079
- US-B1- 6 773 418

## Description

La présente invention aborde le problème général de l'accès, *in vivo,* à l'utérus d'un animal de rente, en vue d'un transfert atraumatique de matériel ou substance à but reproductif, thérapeutique ou diagnostique, ou d'un prélèvement depuis l'utérus, et porte en particulier sur un dispositif permettant un tel accès par la voie vaginale.

On désigne, par l'expression « matériel ou substance à but reproductif » (ou de reproduction), de la semence ou un embryon, le dispositif de transfert en question étant alors un dispositif d'insémination artificielle ou de transfert d'embryon. Le matériel de reproduction pourra être contenu dans une paillette, comme cela est bien connu.

On désigne, par l'expression « matériel ou substance à but thérapeutique », tout agent visant au passage du sujet d'un état pathologique à un état normal, ou à la prévention d'un état pathologique. On peut ainsi mentionner, comme exemple, des antibiotiques intra-utérins profonds, des solutions antiseptiques ou des solutions de lavage isotoniques.

On désigne, par l'expression « matériel ou substance à but diagnostique », tout agent permettant ou aidant un praticien à établir un diagnostic. On peut ainsi mentionner, comme exemple, des substances de marquage, telles que le bleu de méthylène.

On désigne, par l'expression « prélèvement depuis l'utérus », tout prélèvement de matière ou substance à partir de l'utérus, afin de réaliser des analyses telle que la cytologie, une biopsie, etc.

La présente invention s'applique aux animaux de rente, en particulier les bovins, les caprins, et plus généralement aux ongulés.

Si l'on prend l'exemple des bovins, afin d'accéder à l'utérus d'un bovin en vue d'un transfert de matériel ou substance à but reproductif, thérapeutique ou diagnostique, ou d'un prélèvement depuis l'utérus, il est actuellement généralement connu de passer par la voie transrectale.

Cette méthode, illustrée sur les Figures la à 1c, consiste, pour un opérateur, à passer l'un de ses bras dans le rectum Re du bovin, afin de réaliser un cathéterisme du col de l'utérus, le cathéterisme consistant à saisir le col de l'utérus Cu et les cornes utérines Co, et d'aligner le col de l'utérus Cu avec un cathéter T de transfert ou de prélèvement tenu par l'opérateur dans son autre main, le cathéter T étant d'abord inséré dans le vagin V, puis dans le col de l'utérus Cu afin que l'extrémité distale de ce dernier se situe dans l'utérus U de l'animal.

Le vagin V comprend en effet, au niveau de sa partie antérieure, de nombreux replis qu'il convient de franchir pour atteindre le col de l'utérus Cu. Le cervix ou col de l'utérus comprend un canal central appelé canal cervical qui est généralement composé de trois anneaux cervicaux A qu'il est nécessaire d'aligner entre eux pour pouvoir insérer le cathéter T de transfert ou de prélèvement jusque dans l'utérus U du bovin.

Cette méthode présente toutefois plusieurs inconvénients.

Elle est complexe et difficile à réaliser, car elle nécessite que l'opérateur manipule le cathéter tout en ayant l'un de ses bras à l'intérieur du rectum de l'animal. Ainsi, il peut être difficile pour l'opérateur à la fois de manipuler les organes internes de l'animal d'une main, et de manipuler le cathéter de l'autre. La difficulté réside particulièrement dans le fait de franchir le col de l'utérus par une manipulation à l'aveugle, c'est-à-dire sans visualiser les organes de l'animal, l'opérateur se fiant uniquement à son sens du toucher.

De par ces manipulations complexes, un inséminateur réalisant une manipulation transrectale peut souffrir de troubles musculo-squelettiques dus en particulier aux traumatismes subis au niveau des épaules.

Cette méthode présente en outre un risque de blessure du bovin, généralement par lacération ou perforation transrectale due à une manipulation trop brutale des organes génitaux par voie transrectale, ou encore par détérioration des parois du vagin et/ou de l'utérus par le cathéter de transfert ou de prélèvement, ce qui peut dans certains cas conduire à une contamination par introduction dans l'utérus de substances provenant du vagin, et fait courir un risque d'infection au bovin.

En effet, il est nécessaire, lors de l'utilisation de tout dispositif de transfert de matériel ou substance à but reproductif, thérapeutique ou diagnostique, de ne pas blesser l'animal. Par exemple dans le cas de l'insémination artificielle, le traumatisme associé à une blessure engendrera une réaction pro-inflammatoire locale qui perturbe le phénomène d'ovulation puis d'implantation de l'embryon, ce qui limitera les chances de succès de fécondation. Il convient donc d'éviter que le dispositif soit susceptible, par ses dimensions et/ou son fonctionnement, de blesser l'animal. En d'autres termes, le dispositif doit être atraumatique.

Pour les raisons ci-dessus, la méthode par voie transrectale nécessite une certaine expérience et elle est donc actuellement réalisée généralement par des inséminateurs spécialisés, et reste trop complexe pour être réalisée par les éleveurs eux-mêmes.

La présente invention vise à résoudre les problèmes rencontrés avec les techniques de transfert ou de prélèvement classiques décrites ci-dessus en proposant un dispositif pour accéder à l'utérus d'un animal de rente femelle par voie vaginale, en vue d'un transfert de matériel ou substance vers l'utérus ou d'un prélèvement depuis l'utérus, et pour réaliser ledit transfert ou prélèvement, le dispositif évitant le passage par voie transrectale, protégeant l'extrémité du cathéter de transfert ou de prélèvement, étant atraumatique pour l'animal, et évitant de créer des traumatismes musculo-squelettiques chez l'inséminateur.

Le brevet américain US4474576 divulgue un dispositif pour accéder à l'utérus d'un animal de rente femelle par voie vaginale tel que défini dans le préambule de la revendication 1.

La présente invention a donc pour objet un dispositif pour accéder à l'utérus d'un animal de rente femelle, en particulier un bovin, par voie vaginale, en vue d'un transfert de matériel ou substance à but reproductif, thérapeutique ou diagnostique, ou d'un prélèvement depuis l'utérus, et pour réaliser ledit transfert ou prélèvement, le dispositif comprenant un ensemble corps, solidaire de moyens de préhension par un opérateur à son extrémité proximale et ayant une extrémité distale, un premier passage traversant le dispositif et débouchant aux extrémités proximale et distale, de façon à établir une voie d'accès entre l'intérieur et l'extérieur de l'animal, pour permettre le transfert de matériel ou substance à but reproductif, thérapeutique ou diagnostique, ou le prélèvement, l'ensemble corps est sensiblement droit et rigide pour permettre son introduction dans le vagin de l'animal et qu'est prévue à l'extrémité distale une cupule dont l'ouverture est orientée à l'opposé de l'extrémité proximale de l'ensemble corps, une saillie centrale s'étendant à partir du fond de la cupule et ayant un trou traversant dont une extrémité est en communication avec le premier passage traversant de l'ensemble corps, la cupule présentant en outre un orifice en communication avec une extrémité distale d'un second passage traversant prévu dans l'ensemble corps et dont l'autre extrémité, proximale, est configurée pour être reliée à des moyens d'aspiration configurés pour créer dans la cupule une dépression permettant de plaquer la cupule contre le fornix de l'animal, le premier passage formant alors une voie d'accès à l'utérus depuis l'extérieur de l'animal, le dispositif étant caractérisé par le fait que la cupule comprend également un second trou apte à recevoir des moyens de vision ayant un champ de vision orienté dans la direction distale du dispositif pour permettre à un opérateur de visualiser l'environnement devant la cupule.

Le dispositif selon la présente invention permet donc de créer un passage pour un organe de transfert ou de prélèvement dans l'utérus de l'animal, en permettant à l'opérateur de solidariser le col de l'utérus avec le dispositif d'une manière très aisée grâce à la liaison créée entre la cupule et le fornix et grâce aux moyens de vision.

L'orifice en communication avec le second passage traversant est, de préférence, situé dans le fond de la cupule.

Selon un mode de réalisation particulier, les moyens de vision comprennent une caméra ayant un objectif reçu dans le second trou, une source d'éclairage et des moyens de transmission des images capturées par la caméra à un dispositif de visualisation ayant un écran permettant à l'opérateur d'observer lesdites images capturées.

La caméra peut être une caméra endoscopique intégrant la source d'éclairage, lesdits moyens de transmission étant constitués par le corps de la caméra endoscopique, lequel corps s'étend le long de l'ensemble corps jusqu'à la région de l'extrémité proximale de ce dernier, où la caméra endoscopique est reliée au dispositif de visualisation.

En variante, lesdits moyens de transmission consistent en au moins un circuit électronique reçu dans l'ensemble corps et relié à la caméra, l'au moins un circuit électronique étant configuré pour transmettre de manière sans fil les images capturées par la caméra au dispositif de visualisation. Avantageusement, la cupule pourra être transparente ou translucide et comprendre, sur sa partie arrière, des diodes électroluminescentes disposées de façon à éclairer dans la direction distale du dispositif, le faisceau lumineux des diodes traversant la cupule transparente et les diodes constituant ainsi la source d'éclairage pour la caméra.

De préférence, la cupule est reliée de façon amovible à l'ensemble corps. S'il le souhaite, l'opérateur pourra ainsi changer de cupule une fois la précédente souillée. L'opérateur pourra aussi choisir de ne pas changer de cupule entre les animaux, auquel cas une protection sanitaire telle que la chemise décrite ci-après devra être utilisée.

De préférence, pour rendre particulièrement aisé et rapide le changement de cupule, la cupule est reliée de façon amovible à l'ensemble corps par des moyens de type fixation rapide, tels que des moyens de fixation par emboîtage élastique réversible.

Selon un mode de réalisation préféré, le dispositif selon la présente invention comprend une pièce de fixation solidaire de l'ensemble corps et comprenant une région distale qui s'étend à partir de l'ensemble corps, la cupule présentant, côté proximal, un renfoncement configuré pour recevoir ladite région distale, l'une ou l'autre, ou les deux, parmi la cupule et ladite région distale présentant les moyens de type fixation rapide, la pièce de fixation présentant en outre un passage traversant en communication avec le premier passage traversant de l'ensemble corps et avec le trou traversant de la saillie de la cupule lorsque cette dernière est fixée à la pièce de fixation.

Comme indiqué ci-dessus, les moyens de vision peuvent comprendre une caméra ayant un objectif reçu dans le second trou de la cupule, une source d'éclairage et des moyens de transmission des images capturées par la caméra à un dispositif de visualisation ayant un écran. Les moyens de vision peuvent être une caméra endoscopique comme décrit ci-dessus. Selon une variante avantageuse, lesdits moyens de transmission consistent en au moins un circuit électronique reçu dans l'ensemble corps et relié à la caméra, l'au moins un circuit électronique étant configuré pour transmettre de manière sans fil les images capturées par la caméra au dispositif de visualisation, la pièce de fixation et la cupule étant transparentes, une source d'éclairage, telle que des diodes électroluminescentes, étant montée à l'intérieur de la pièce de fixation de façon à éclairer dans la direction distale du dispositif.

Selon un mode de réalisation particulier, l'ensemble corps est de forme cylindrique et comprend une partie corps solidaire des moyens de préhension, la partie corps étant, de préférence, formée en matière plastique telle que du polychlorure de vinyle.

L'orifice de la saillie de la cupule peut être fermé par un capuchon en matière élastomère solidaire de la saillie, le capuchon protégeant l'organe de transfert ou de prélèvement par rapport à d'éventuelles sécrétions de l'animal après retrait du bouchon, le capuchon ayant une fente centrale en croix permettant à un organe de transfert ou de prélèvement de passer à travers celui-ci lorsque ledit organe est poussé par un opérateur, et donc de dépasser de la saillie.

Ainsi, l'organe de transfert ou de prélèvement est davantage protégé des sécrétions vaginales du bovin.

Les moyens de préhension peuvent comprendre une poignée et un corps, les moyens de préhension combinés à l'ensemble corps ayant une forme de pistolet. La forme de pistolet permet une préhension aisée du dispositif par un opérateur.

Les moyens d'aspiration peuvent comprendre une pompe manuelle ayant un piston et une chambre, une partie de la poignée des moyens de préhension étant montée pivotante par rapport au corps des moyens de préhension et étant reliée au piston, ce par quoi un déplacement de la poignée actionne le piston et crée une aspiration dans la chambre.

Ainsi, en actionnant plusieurs fois de suite la poignée, un opérateur peut créer une dépression suffisante pour plaquer la cupule contre le col de l'utérus. En outre, les moyens d'aspiration étant intégrés au dispositif, il n'est pas nécessaire d'utiliser des moyens d'aspiration annexes.

En variante, les moyens d'aspiration peuvent être distincts du dispositif, et être par exemple formés par une pompe à vide actionnable manuellement ou au pied.

Selon un mode de réalisation particulier, l'ensemble corps est de forme cylindrique, et comprend une partie corps solidaire des moyens de préhension et une partie de tête formant l'extrémité distale dudit ensemble corps, la partie de tête étant reliée à la partie corps par une liaison pivot le long d'un diamètre de l'ensemble corps, le dispositif comprenant en outre des moyens d'actionnement permettant d'orienter la partie de tête entre une position dite droite dans laquelle la partie corps et la partie de tête sont alignés sur une même droite, et une position dite inclinée dans laquelle la partie corps et la partie de tête forment entre elles un angle non nul, de préférence compris entre 0° et 35°.

L'entrée du col de l'utérus n'est généralement pas alignée avec le vagin de l'animal. Dans l'état antérieur de la technique, l'opérateur passait par la voie transrectale pour manipuler les organes et les aligner. Dans le cas du présent dispositif, une inclinaison de la partie distale permet d'éviter la manipulation transrectale lorsque les organes ne sont pas alignés.

De préférence, les moyens d'actionnement permettant d'orienter la partie de tête comprennent un étrier, un câble et un ressort, l'étrier étant monté pivotant sur et par rapport aux moyens de préhension, le câble reliant l'étrier et un premier point de liaison formé dans la partie de tête, et le ressort reliant la partie de tête et un second point formé dans la partie corps, les premier et second points étant situés de part et d'autre d'un plan imaginaire comprenant la liaison pivot et coupant la partie corps dans le sens de la longueur en deux parties égales, de telle sorte que la partie de tête est placée en position inclinée lors d'un pivotement de l'étrier vers l'extrémité proximale du dispositif, par traction sur le câble, et revient en position droite sous l'action du ressort lorsque l'étrier est relâché.

Ainsi, les moyens d'actionnement peuvent être manipulés d'une seule main par l'opérateur, ladite main tenant le dispositif.

L'extrémité distale de la cupule pourra être fermée de manière sélective par un bouchon en matière élastomère, apte à être solidarisé avec la cupule par insertion en force, le bouchon protégeant l'animal lors de l'insertion contre les bords de la cupule qui peuvent traumatiser l'animal, et protégeant l'intérieur de la cupule par rapport à d'éventuelles sécrétions de l'animal, le bouchon ayant sur sa face externe un trou traversant à l'aide duquel un fil est rendu solidaire du bouchon, le fil étant de longueur suffisante pour dépasser à l'extérieur de l'animal après insertion du dispositif dans le vagin de l'animal, et le fil permettant, par traction sur ce dernier, de retirer le bouchon lorsque nécessaire, afin de libérer le passage pour l'organe de transfert ou de prélèvement.

La caractéristique atraumatique est assurée par le bouchon, car sa forme, couplée à celle de la cupule et du reste du dispositif, permet l'insertion dans le vagin sans risquer d'endommager les parois de ce dernier.

En pratique, il peut arriver que le champ de vision des moyens de vision soit obstrué par des sécrétions de plus grande dimension que l'orifice configuré pour être relié aux moyens d'aspiration permettant de plaquer la cupule contre le fornix de l'animal. Ainsi, par exemple, on rencontrera souvent des glaires, notamment des glaires de chaleur dans le cas de l'insémination artificielle. Pour surmonter cette difficulté, selon la présente invention la cupule peut comprendre avantageusement un trou traversant d'aspiration de glaires, dont une extrémité distale débouche côté ouverture de la cupule et dont l'extrémité proximale est configurée pour être reliée à une extrémité distale d'une gaine d'aspiration dont l'extrémité proximale est configurée pour être reliée à des moyens d'aspiration de glaires eux-mêmes configurés pour créer une dépression permettant d'aspirer les éventuelles glaires depuis l'ouverture de la cupule vers l'extérieur du dispositif en passant par le trou traversant d'aspiration de glaires et la gaine d'aspiration.

Les moyens d'aspiration de glaires pourront prendre toute forme appropriée, comme par exemple des moyens distincts du dispositif, tels qu'une pompe à vide actionnable manuellement ou au pied.

Ladite gaine d'aspiration pourra s'étendre à l'intérieur de ou le long de la partie corps de l'ensemble corps, et ainsi, selon la présente invention, est considérée comme étant dans l'ensemble corps.

Est également divulguée, mais non revendiquée, une cupule pour un dispositif tel que défini ci-dessus.

La cupule peut comprendre en outre une chemise sanitaire, sensiblement tubulaire et fermée à son extrémité distale, enroulée sur elle-même a son extrémité proximale, et disposée d'une manière enveloppant la cupule et de façon à être apte à être déroulée sur l'ensemble corps d'un dispositif tel que défini ci-dessus, après que la cupule a été fixée sur ledit dispositif.

Pour mieux illustrer l'objet de la présente invention, on va en décrire ci-après, à titre indicatif et non limitatif, des modes de réalisation particuliers avec référence au dessin annexé.

Sur ce dessin :
- les Figures la à 1c sont des illustrations des différentes étapes de la méthode d'insertion d'un organe de transfert ou de prélèvement dans l'utérus d'un bovin par la voie transrectale, selon l'état antérieur de la technique ;
- les Figures 2 et 3 sont des vues en perspective avant et arrière, respectivement, du dispositif selon un premier mode de réalisation de la présente invention ;
- les Figures 4 et 5 sont des vues en coupe du dispositif selon le premier mode de réalisation de la présente invention, respectivement en position droite et inclinée ;
- la Figure 6 est une vue de l'intérieur de la cupule du dispositif selon le premier mode de réalisation de la présente invention ;
- les Figures 7 et 8 sont des vues de détail, respectivement en perspective et de face, de la pièce de jonction du dispositif selon le premier mode de réalisation de la présente invention ;
- les Figures 9 et 10 sont des vues de détail, respectivement en perspective et en coupe de profil, de l'extrémité distale du dispositif selon le premier mode de réalisation de la présente invention, le bouchon étant monté à ladite extrémité distale ;
- la Figure 11 est une vue de profil du dispositif selon le premier mode de réalisation de la présente invention, dans sa position initiale avant insertion dans le vagin du bovin ;
- la Figure 12 est une vue de profil du dispositif selon le premier mode de réalisation de la présente invention, dans sa position de transfert de matériel ou substance ou de prélèvement depuis l'utérus ;
- la Figure 13a est une vue en coupe du dispositif selon le premier mode de réalisation de la présente invention inséré dans le vagin d'un bovin, avec la cupule plaquée contre le col de l'utérus et l'extrémité distale de l'organe de transfert et de prélèvement dans l'utérus ;
- la Figure 13b est une vue de détail de la Figure 13a, montrant l'extrémité distale du dispositif selon le premier mode de réalisation de la présente invention ;
- la Figure 14 est une vue en coupe et de profil du dispositif selon un second mode de réalisation de la présente invention ;
- les Figures 15 et 16 sont des vues en perspective de la cupule du dispositif selon le second mode de réalisation de la présente invention, la cupule étant vue respectivement depuis l'avant et depuis l'arrière ;
- la Figure 17a est une vue en perspective de la cupule des Figures 15 et 16, enveloppée d'une chemise sanitaire ;
- la Figure 17b est une vue en perspective du pistolet, sur lequel la chemise de la Figure 17a a été déroulée ;
- les Figures 18 et 19 sont des vues analogues respectivement aux Figures 14 et 6, montrant une variante du dispositif selon le second mode de réalisation ; et
- les Figures 20, 21 et 22 sont des vues respectivement de côté, en coupe longitudinale et de dessus, de la région distale d'un dispositif selon un troisième mode de réalisation.

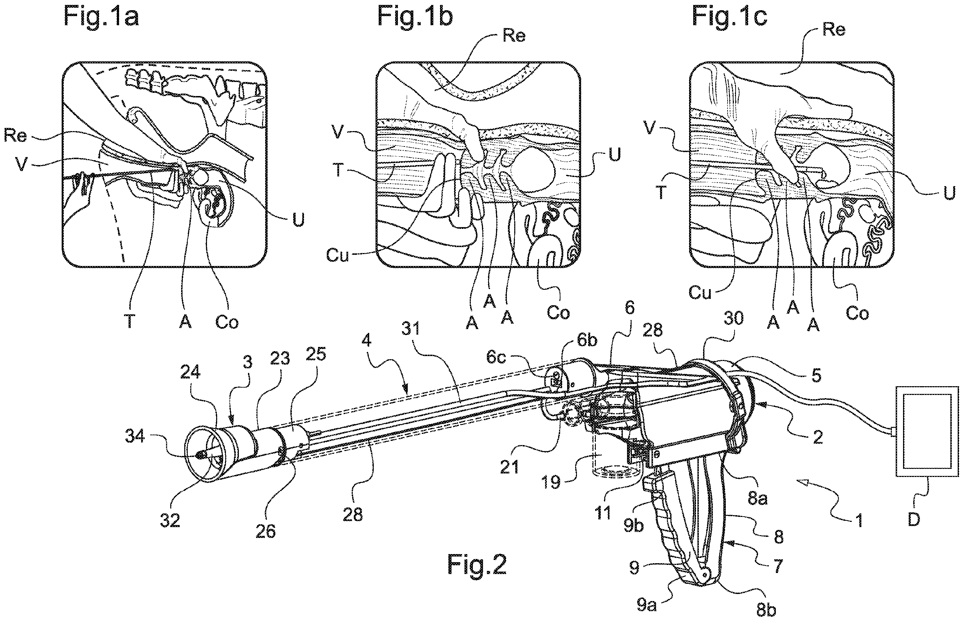

Si l'on se réfère aux Figures 2 à 9, on peut voir qu'un dispositif 1 pour accéder à l'utérus d'un animal de rente femelle par voie vaginale selon un mode de réalisation particulier de la présente invention comprend un corps proximal 2 et une partie distale 3 reliés par un corps tubulaire 4, le dispositif 1 étant globalement en forme de pistolet.

Le corps proximal 2 comprend une partie cylindrique creuse 5 ayant, dans sa région distale supérieure, une saillie 6 dont l'extrémité distale est cylindrique et a un diamètre très légèrement inférieur au diamètre interne du corps tubulaire 4 pour que ladite extrémité distale soit insérée dans le corps tubulaire 4 et rendue solidaire de celui-ci.

Le corps tubulaire 4 est en matière plastique, telle que du PVC (polychlorure de vinyle).

Le corps proximal 2 comprend également une poignée 7, disposée de manière diamétralement opposée à la saillie 6, la poignée 7 comprenant une partie fixe 8 sensiblement droite, dont une première extrémité 8a est fixée à l'extrémité proximale de la partie cylindrique 5 et s'étendant radialement à partir de celle-ci.

La poignée 7 comprend également une partie mobile 9 sensiblement droite, dont une première extrémité 9a est montée pivotante sur la seconde extrémité 8b de la partie fixe 8. La seconde extrémité 9b de la partie mobile 9 est solidaire d'une première extrémité 10a d'un coulisseau 10 sensiblement parallélépipédique et monté coulissant dans le sens de sa longueur dans une rainure 11 formée sur le corps cylindrique 5 et dans la direction de la longueur de celui-ci.

Ainsi, la seconde extrémité 10b du coulisseau 10 est située de manière plus proximale que ladite première extrémité 10a du coulisseau 10. La seconde extrémité 10b présente un coude 12 ayant une partie droite 13 se prolongeant radialement vers l'axe de révolution de la partie cylindrique 5 au niveau duquel celle-ci est solidaire d'un piston 14.

Le piston 14 comprend une tête 15 et une tige 16 solidaire de la tête 15 et dirigée vers le côté proximal du dispositif 1. Ainsi, l'extrémité de la partie droite 13 est fixée à l'extrémité proximale de la tige 16.

Le piston 14 est disposé dans la partie interne creuse de la partie cylindrique 5 du corps proximal 2, et l'espace interne délimité par la surface interne de la partie cylindrique 5 et par la tête de piston 15 forme une chambre 17.

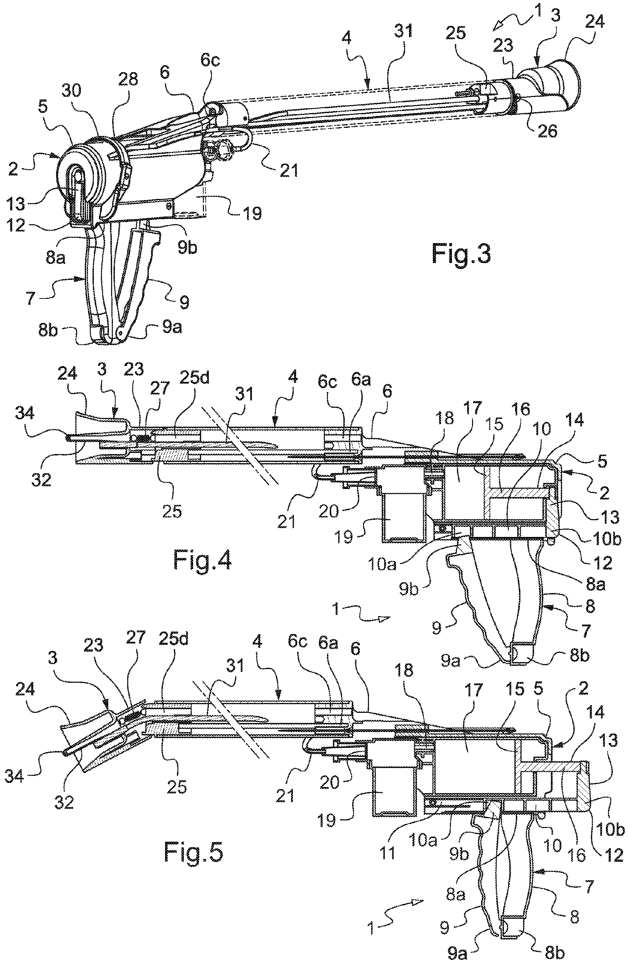

Un ressort (non représenté) s'étend entre la seconde extrémité 9b de la partie mobile 9 et l'extrémité distale de la rainure 11, le ressort permettant de ramener la partie mobile 9 en position initiale, comme représenté sur la Figure 4, après que la seconde extrémité 9b a été déplacée vers le côté proximal du dispositif 1, comme représenté sur la Figure 5.

La chambre 17, à son extrémité distale, est reliée de manière fluidique par un canal 18 à un réservoir 19 solidaire du corps proximal 2.

Une première extrémité d'une gaine d'aspiration 21 est reliée à la partie distale 3, la gaine 21 passant à l'intérieur du corps tubulaire 4.

Le réservoir 19 présente un orifice de sortie 20 relié à une première branche d'une vanne quart de tour 22 en T, la deuxième branche du T étant reliée à l'autre extrémité de la gaine 21 et la troisième branche du T étant reliée à un orifice débouchant vers l'extérieur.

La vanne 22 est actionnable entre une première position, dite d'aspiration, dans laquelle le réservoir 19 et la gaine 21 sont reliés fluidiquement entre eux et ne sont pas reliés fluidiquement à l'orifice débouchant vers l'extérieur, ce qui permet d'exercer une aspiration au niveau de la première extrémité de la gaine 21 lors de l'actionnement du piston 14, et une seconde position, dite de mise à l'air, dans laquelle le réservoir 19, la gaine 21 et l'orifice débouchant vers l'extérieur sont reliés fluidiquement, ce qui permet de ramener la première extrémité de la gaine 21 à la pression atmosphérique.

La partie distale 3 comprend une partie corps 23 proximale et une partie cupule 24 distale.

La partie distale 3 peut être réalisée, par exemple, en matière plastique transparente.

La partie cupule 24 est disposée avec son extrémité ouverte orientée vers le côté distal du dispositif 1. La seconde extrémité de la gaine 21 est en particulier reliée à un orifice 24a formé dans le fond de la partie cupule 24 et débouchant vers l'extérieur.

La partie corps 23 est cylindrique et creuse, et est reliée au corps tubulaire 4 par l'intermédiaire d'une pièce de jonction 25.

La région d'extrémité proximale de la pièce de jonction 25 est cylindrique, a un diamètre légèrement inférieur à celui du corps tubulaire 4 et est insérée dans ce dernier de manière fixe.

Ainsi, le corps tubulaire 4 s'étend entre la saillie 6 et la pièce de jonction 25.

La région d'extrémité distale de la pièce de jonction 25 est cylindrique, a un diamètre légèrement inférieur à celui de la partie corps 23 et est entourée par cette dernière.

La partie corps 23 et la pièce de jonction 25 sont reliées par une liaison pivot 26 située sur un diamètre de la pièce de jonction 25, la liaison 26 étant sensiblement horizontale lorsque la poignée 7 du corps proximal 2 est dirigée vers le bas.

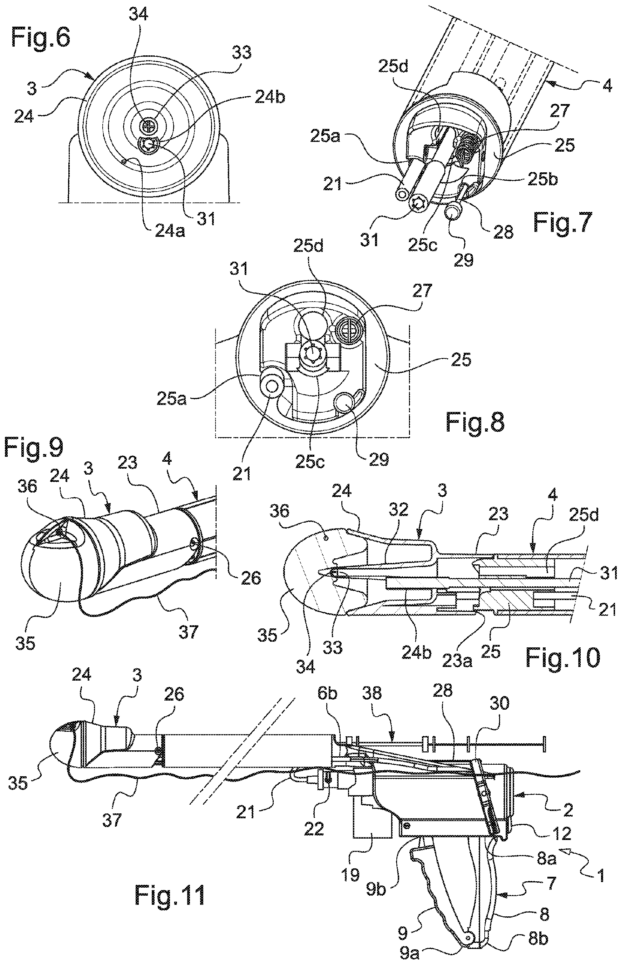

De plus, lorsque la poignée 7 du corps proximal 2 est dirigée vers le bas, on peut voir en particulier sur la Figure 10 que la partie corps 23 entoure la pièce de jonction 25 uniquement sur la partie supérieure de celle-ci. Une échancrure 23a est en effet formée sur la partie corps 23. Cette échancrure 23a permet à la partie distale 3, lors de son pivotement autour de la liaison pivot 26, comme représenté sur la Figure 5, de ne pas être bloquée par la pièce de jonction 25 et de pouvoir être ainsi librement inclinée.

La pièce de jonction 25 comprend quatre orifices 25a, 25b, 25c, 25d. La gaine 21 passe dans l'orifice 25a, les éléments passant dans les autres orifices seront détaillés plus loin dans la description.

Un ressort 27 relie la partie corps 23 et la pièce de jonction 25 et est disposé d'un côté d'un plan imaginaire auquel appartiennent l'axe de la liaison pivot 26 et l'axe longitudinal du corps tubulaire 4.

De l'autre côté dudit plan, une première extrémité d'un câble 28 est rendue solidaire de la partie corps 23 par l'intermédiaire d'une pièce de blocage 29, solidaire du câble 28. La pièce de blocage 29 est montée à force dans une cavité (non représentée) formée à l'intérieur de la partie corps 23.

L'autre extrémité du câble 28 est fixée en partie supérieure d'un étrier 30 enveloppant le corps proximal 2, et dont la partie inférieure est montée pivotante sur les parois extérieures de la partie du corps proximal 2 définissant la rainure 11.

Le câble 28 passe par un orifice 6a de la saillie 6 du corps proximal 2, par le corps tubulaire 4 et par l'orifice 25b de la pièce de jonction 25.

Le dispositif 1 comprend également une caméra 31 de type endoscope, à source d'éclairage intégrée, dont l'objectif affleure au niveau d'un orifice 24b formé dans le fond de la cupule, et dont l'autre extrémité est reliée à des moyens d'affichage (non représentés), la caméra passant par l'orifice 25c de la pièce de jonction 25, par le corps tubulaire 4 et par un orifice 6b de la saillie 6 du corps proximal 2, pour être reliée enfin à un dispositif de visualisation D représenté schématiquement, tel qu'un moniteur, un ordinateur, une tablette électronique, un téléphone intelligent, etc.

L'orifice 25c est formé au centre de la pièce de jonction 25.

La partie cupule 24 comprend également une saillie 32, formée au centre du fond de la partie cupule 24 et dirigée vers le côté distal de celle-ci, un trou traversant 33 s'étendant d'une extrémité à l'autre de la saillie 32 et débouchant sur l'extérieur au sommet de cette dernière.

Le trou traversant 33 est aligné avec le trou 25d de la pièce de jonction 25, le trou 25d étant formé du même côté du plan imaginaire coupant la pièce 25 que le ressort 27, et est situé sur le diamètre perpendiculaire à l'axe de la liaison pivot 26.

En d'autres termes, l'orifice 25d est juste au-dessus de l'orifice 25c, lorsque le pistolet est tenu avec la poignée 7 dirigée vers le bas.

Ainsi, l'axe de révolution de la partie cupule 24 est légèrement décalé vers le haut par rapport à celui de la partie corps 23.

L'orifice 25d est également aligné avec un orifice 6c de la saillie 6, ce qui forme un chemin rectiligne pour le passage d'un organe de transfert ou de prélèvement, dont l'utilisation sera décrite plus loin dans la description.

Le trou traversant 33 est fermé par un capuchon à fentes 34. Le capuchon 34 est en matière élastomère, a une forme d'ogive et présente une fente en croix dont le centre correspond au sommet de l'ogive. Il permet d'éviter les interactions entre l'organe de transfert ou de prélèvement et l'extérieur lorsque ledit organe ne dépasse pas par le trou traversant 33.

Le capuchon 34 est apte à laisser passer ledit organe par passage par les fentes.

Le dispositif 1 comprend également un bouchon 35 en matériau de type caoutchouc inséré de manière amovible dans la partie cupule 24. Le bouchon 35 comprend un trou 36 dans lequel passe un fil 37, le fil 37 permettant, par traction sur ce dernier, de déloger le bouchon 35 de la partie cupule 24.

Le bouchon 35 a une forme arrondie à son extrémité distale, et son diamètre à la base est sensiblement identique à celui de la partie cupule 24.

Ainsi, l'ensemble formé par le bouchon 35 et la partie cupule 24 ne présente pas de bord anguleux, et ne présente donc pas de risque de blessure pour l'animal, et a une forme facilitant le passage du dispositif 1 au travers des plis du vagin de l'animal.

Comme représenté sur les Figures 10 et 11, en utilisation, un opérateur place un organe de transfert ou de prélèvement 38 dans le passage du dispositif 1 prévu à cet effet, jusqu'à ce que l'extrémité distale dudit organe soit située dans la saillie 32 de la cupule 24, à proximité du capuchon à fente 34.

Le dispositif 1 est alors en position initiale, comme représenté sur la Figure 11, avec la partie distale 3 alignée avec le corps tubulaire 4, et le bouchon 35 étant présent.

L'opérateur insère ensuite le dispositif 1 par son extrémité distale dans le vagin V d'un animal de rente femelle jusqu'à ce que le bouchon 35 vienne au contact du col de l'utérus Cu de l'animal.

Le bouchon 35 est alors retiré par l'opérateur, en tirant sur le fil 37 que l'opérateur aura pris soin de faire passer le long du dispositif 1 jusqu'à l'extérieur.

Une fois le bouchon 35 retiré, l'opérateur peut alors visualiser le col de l'utérus Cu par l'intermédiaire de la caméra 31, et aligner la saillie 32 avec le col Cu. On souligne ici que la longueur de la saillie 32 est supérieure à la profondeur de la cupule 24, en d'autres termes la saillie 32 dépasse du bord périphérique libre de la cupule 24, de telle sorte que l'extrémité libre de la saillie 32 entre en premier dans le col Cu et permet de centrer correctement la cupule 24 par rapport au col Cu.

Il est possible pour l'opérateur d'incliner la partie distale 3, par actionnement de l'étrier 30, si la configuration des organes du bovin le nécessite.

Ainsi, comme on peut le voir sur la Figure 4, lorsque la partie supérieure de l'étrier 30 est déplacée vers le côté proximal du dispositif 1, une traction est exercée sur le câble 30, ce qui a pour effet de faire pivoter la partie distale 3 vers le bas à partir de sa position initiale, à savoir alignée avec le corps tubulaire 4.

L'opérateur peut ainsi plus facilement aligner la saillie 32 avec le col de l'utérus Cu.

Une fois que le col de l'utérus Cu et la saillie 32 sont globalement alignés, l'opérateur place la partie cupule 24 contre le fornix du le col Cu, et actionne la poignée 9 afin de déplacer le piston 14 et créer une dépression dans la chambre 17.

Cette dépression, lorsque la vanne quart de tour 22 est dans la première position, crée une aspiration du col de l'utérus Cu dans la partie cupule 24, et permet ainsi de solidariser la partie cupule 24 avec le col de l'utérus Cu pour faciliter le passage de l'organe de transfert ou de prélèvement 38. L'opérateur peut en particulier déplacer le dispositif 1 afin d'aligner les anneaux A du col de l'utérus Cu.

Une fois que la partie cupule 24 est rendue solidaire du col de l'utérus Cu, il n'est plus nécessaire d'orienter la partie distale 3 du dispositif 1. Ainsi, lorsque la force agissant sur l'étrier 30 est relâchée, le ressort 27 exerce une force qui ramène la partie distale 3 dans sa position initiale, et par traction sur le câble 28, replace également l'étrier 30 dans sa position initiale.

Durant l'aspiration, des sécrétions de l'animal peuvent être aspirées par le dispositif 1. Ces sécrétions passent par l'orifice d'aspiration 24a puis par la gaine d'aspiration 21 et la vanne quart de tour 22, puis chutent par gravité dans le récipient de récupération 19. Le canal 18 reliant le récipient 19 à la chambre 17 est situé en partie haute du récipient 19, de telle sorte que les sécrétions ne peuvent pas aller dans la chambre 17, ce qui évite ainsi une mauvaise performance d'aspiration.

Le maintien de l'aspiration permet à un opérateur de pouvoir manipuler le col de l'utérus avec le dispositif. Un autre avantage, secondaire, est qu'il permet d'éviter que des sécrétions vaginales de l'animal ne pénètrent dans l'utérus U, ce qui évite ainsi une contamination de l'utérus U par des bactéries qui peuvent être présentes dans ces substances.

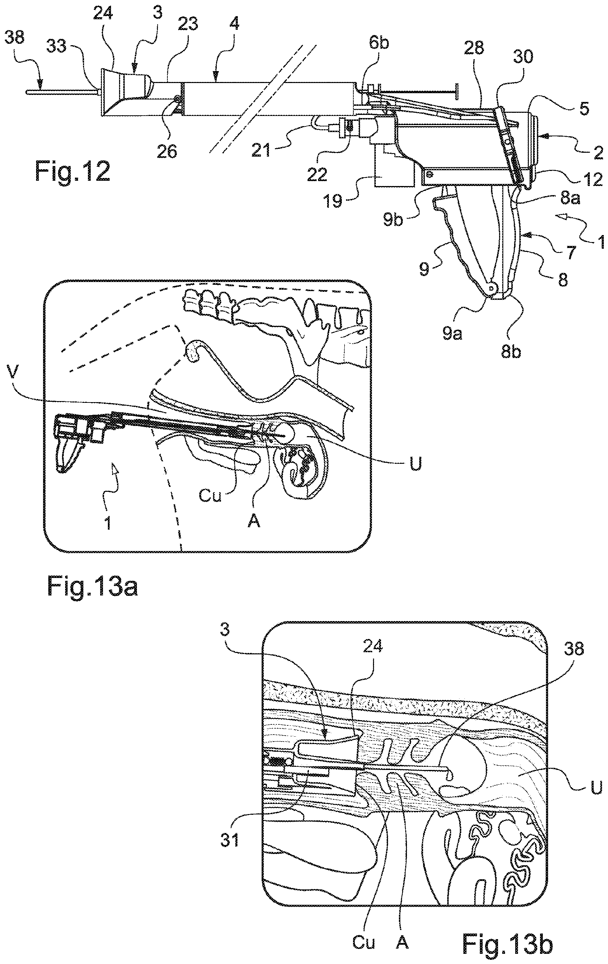

L'opérateur pousse ensuite l'organe de transfert et de prélèvement 38 pour passer le capuchon 34, jusqu'à ce que l'extrémité distale dudit organe se situe dans l'utérus. Cette position est représentée sur les Figures 13a et 13b.

On peut voir en particulier sur la Figure 13b que la forme de cloche de la partie cupule 24 est prévue pour se conformer à la forme du col de l'utérus Cu, ce qui assure ainsi la solidarisation de la partie cupule 24 et du col de l'utérus Cu.

Une fois l'opération de transfert ou de prélèvement réalisée, l'opérateur retire l'organe de transfert ou de prélèvement du dispositif 1. Il actionne ensuite la vanne quart de tour 22 pour la placer dans la seconde position, dans laquelle le réservoir 19 et la gaine 21 sont alors mis à la pression atmosphérique, ce qui permet de désolidariser la partie cupule 24 du col de l'utérus Cu, et ainsi de retirer le dispositif 1 du bovin.

Le dispositif 1 selon la présente invention permet donc à un opérateur seul, en particulier l'éleveur, d'accéder, de manière simple, à l'utérus d'un animal de rente femelle par voie vaginale, en vue d'un transfert de matériel ou substance à but reproductif, thérapeutique ou diagnostique, ou d'un prélèvement depuis l'utérus, et pour réaliser ledit transfert ou prélèvement.

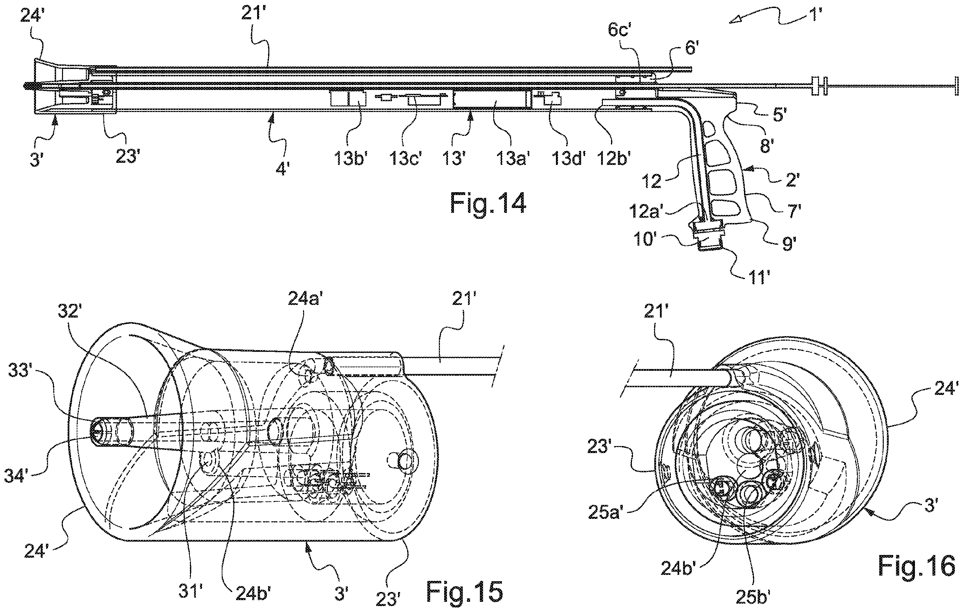

Si l'on se réfère aux Figures 14 à 16, on peut voir que l'on y a représenté un dispositif 1' selon un second mode de réalisation de la présente invention, lequel comprend sensiblement les mêmes éléments que le dispositif 1 décrit ci-dessus. Ainsi, le dispositif 1' comprend un corps proximal 2' et une partie distale 3' reliés par un corps tubulaire 4', le dispositif 1' étant globalement en forme de pistolet.

Le corps proximal 2' comprend une partie de corps 5' sensiblement en forme de demi-cylindre ayant, dans sa région distale supérieure, une saillie 6' dont la région d'extrémité distale est cylindrique et a un diamètre légèrement inférieur au diamètre interne du corps tubulaire 4', ladite région d'extrémité distale étant insérée dans le corps tubulaire 4' et rendue solidaire de celui-ci.

Comme pour le premier mode de réalisation, le corps tubulaire 4' est matière plastique, telle que du PVC (polychlorure de vinyle).

Le corps proximal 2' comprend également une poignée 7', disposée de manière diamétralement opposée à la saillie 6', la poignée 7' ayant une première extrémité 8' fixée à l'extrémité proximale de la partie de corps 5' et s'étendant radialement à partir de celle-ci.

La poignée 7' comprend également, à sa seconde extrémité 9' opposée à la première extrémité 8', un connecteur 10' de type port USB, pouvant être fermé par un bouchon 11'.

Le connecteur 10' est relié à la première extrémité 12a' d'un faisceau 12'. L'autre extrémité 12b' du faisceau 12' est reliée à un ensemble électronique de transmission sans fil 13', ledit ensemble 13' comprenant une batterie 13a' et des cartes électroniques 13b', 13c', 13d' comprenant des puces de transmission de données sans fil.

Le connecteur 10' permet de brancher le dispositif 1' à une source d'alimentation, qui recharge la batterie 13a' lorsque celle-ci est vide.

Une première extrémité d'une gaine d'aspiration 21' est reliée à des moyens d'aspiration (non représentés), tels qu'une pompe à vide manuelle, l'autre extrémité de la gaine 21' étant reliée à la partie distale 3', la gaine 21' s'étendant le long du corps tubulaire 4', sur l'extérieur de celui-ci.

La partie distale 3' comprend une partie corps 23' proximale et une partie cupule 24' distale.

La partie cupule 24' est disposée avec son extrémité ouverte orientée vers le côté distal du dispositif 1'. Ladite autre extrémité de la gaine 21' est en particulier reliée à un orifice 24a' formé dans le fond de la partie cupule 24' et débouchant vers l'extérieur.

La partie corps 23' est cylindrique et creuse, et entoure le corps tubulaire 4'.

Le dispositif 1' comprend également une caméra 31' dont l'objectif affleure au niveau d'un orifice 24b' formé dans le fond de la partie cupule 24', et dont le corps est relié, à son autre extrémité, à l'ensemble de transmission de données sans fil 13', le corps de la caméra 31' passant à l'intérieur du corps tubulaire 4'.

L'ensemble de transmission de données sans fil 13' est configuré pour recevoir les images enregistrées par la caméra 31' et transmettre ces dernières de manière sans fil, de préférence par WiFi, à un dispositif de visualisation distant tel qu'un ordinateur, grâce aux cartes électroniques 13b', 13c', 13d'.

La partie cupule 24' laisse passer au moins une partie de la lumière. Elle peut ainsi être transparente ou translucide par exemple. On pourra par exemple utiliser pour la partie distale 3' du polyéthylène ou du polypropylène.

La partie cupule 24' comprend, dans sa partie proximale, côté opposé au fond de la partie cupule 24' qui est tourné vers l'extérieur, des diodes électroluminescentes (DEL) 25a' et 25b' montées dans des logements cylindriques formés par la partie cupule 24'. Les DEL 25a' et 25b' ont une direction d'éclairage orientée vers l'extrémité distale de la partie cupule 24'.

Les DEL 25a' et 25b' sont alimentées par la batterie 13a' du dispositif 1', et permettent d'éclairer, par transparence de la matière formant la partie cupule 24, le col de l'utérus de l'animal. Ainsi, la lumière procurée par les DEL permet à la caméra 31' de capturer des images de l'environnement devant la cupule 24' pour un placement plus aisé du dispositif 1' contre le fornix du col de l'utérus de l'animal et un alignement plus aisé des anneaux du col de l'utérus.

La partie cupule 24' comprend également une saillie 32', formée au centre du fond de la cupule 24' et dirigée vers le côté distal de celle-ci, la saillie 32' comprenant un trou traversant 33' fermé par un capuchon à fentes 34', de manière analogue au premier mode de réalisation. Comme pour le premier mode de réalisation, l'axe de révolution de la partie cupule 24' est légèrement décalé vers le haut par rapport à celui de la partie corps 23'.

Bien que non représenté, le dispositif 1' pourra également comprendre un bouchon analogue au bouchon 35, avec un fil associé.

Le dispositif 1' selon le second mode de réalisation est utilisé de manière analogue au dispositif 1, à la différence du fait que l'aspiration n'est pas obtenu par un mécanisme de type gâchette, mais par l'actionnement de moyens d'aspiration distincts (pompe à vide manuelle), et du fait que le dispositif 1' est muni d'une électronique lui permettant de transmettre de manière sans fil les images enregistrées par la caméra 31'.

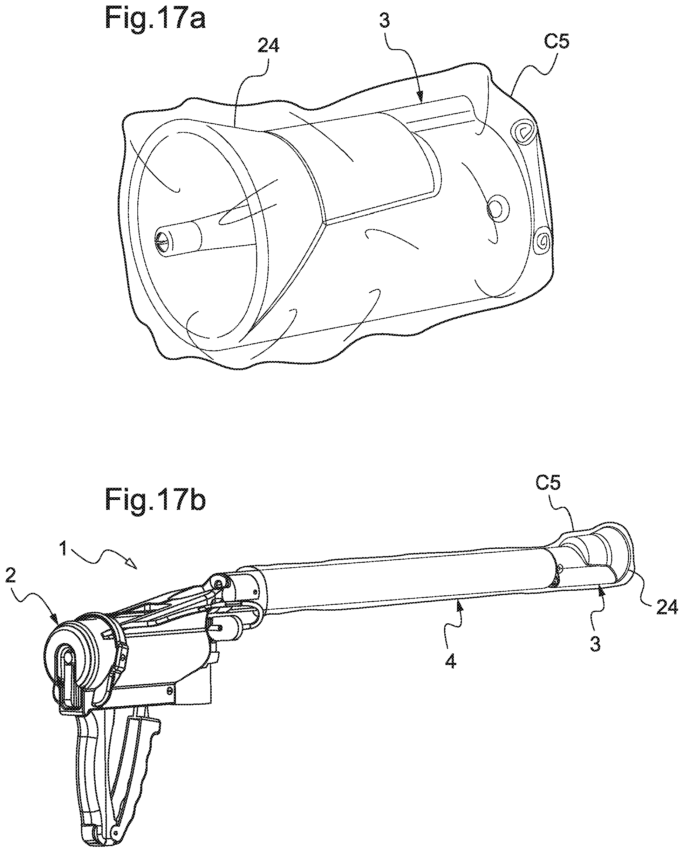

Si l'on se réfère maintenant aux Figure 17a et 17b, on peut voir que l'on y a représenté une cupule 24 enveloppée par une chemise sanitaire CS, la protégeant vis-à-vis de l'extérieur. La chemise sanitaire CS est disposée enroulée sur la cupule 24, comme représenté sur la Figure 17a, et après que la cupule 24 a été emmanchée sur l'ensemble corps 4, la chemise sanitaire CS est déroulée sur l'ensemble corps 4, de façon à protéger la partie distale du dispositif 1, comme représenté sur la Figure 17b. La chemise sanitaire CS peut également être utilisée avec la cupule 24' selon le premier mode de réalisation.

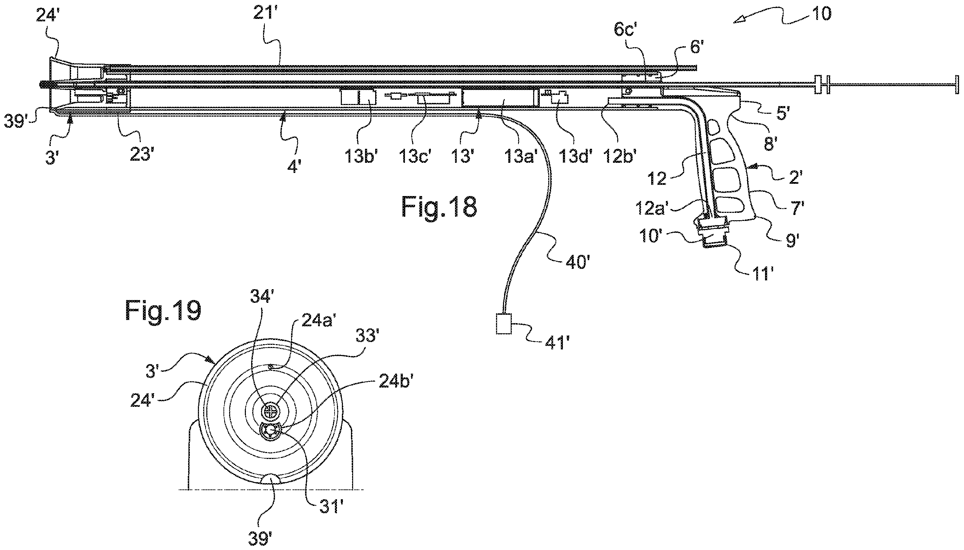

Si l'on se réfère maintenant aux Figures 18 et 19, on peut voir que l'on y a représenté une variante du dispositif 1' du second mode de réalisation, qui diffère de ce dernier uniquement en ce qu'il comporte des moyens permettant d'aspirer les éventuelles glaires qui pourraient obstruer le champ de vision de la caméra 31'.

Ces moyens comprennent un trou traversant d'aspiration de glaires 39' qui débouche, à son extrémité distale, dans l'intérieur de la cupule 24', et, à son extrémité proximale, sur l'extérieur de la cupule 24', par exemple au niveau d'une partie tubulaire formant raccord, à laquelle l'extrémité distale d'une gaine d'aspiration 40' s'étendant le long du corps 4' peut être reliée de manière étanche, par exemple par insertion à force, tandis que l'extrémité proximale de la gaine d'aspiration 40' est reliée à des moyens d'aspiration 41' représentés très schématiquement, et qui pourront par exemple être une pompe à vide à actionnement manuel ou au pied.

On comprend aisément que l'actionnement des moyens d'aspiration permet d'aspirer des éléments qui se trouveraient dans la cupule, et notamment des glaires. A cet effet, le trou traversant d'aspiration de glaires 39' sera dimensionné pour être aptes à aspirer des glaires (à savoir qu'il sera suffisamment grand).

En pratique, lorsque l'opérateur ne voit plus l'intérieur de l'animal lors de l'introduction du dispositif 1', ou lors de l'opération de transfert de matériel ou substance ou de prélèvement, car des glaires obstruent le champ de vision de la caméra 31', il lui suffit d'actionner les moyens d'aspiration de glaires 41', qui aspireront alors lesdits glaires, dégageant ainsi le champ de vision de la caméra 31'.

Dans l'exemple représenté, l'orifice 24a' et le trou 39' sont diamétralement opposés. Bien entendu, la présente invention n'est pas limitée à ce positionnement, et l'on pourra placer l'orifice 24a' et le trou 39' dans n'importe quelles positions. Par exemple, il sera avantageux de les prévoir tous les deux à proximité l'un de l'autre, côté inférieur de la cupule 24' lorsque l'on regarde les Figures 18 et 19, de telle sorte que les gaines d'aspiration 21' et 40' s'étendent toutes deux à proximité l'une de l'autre.

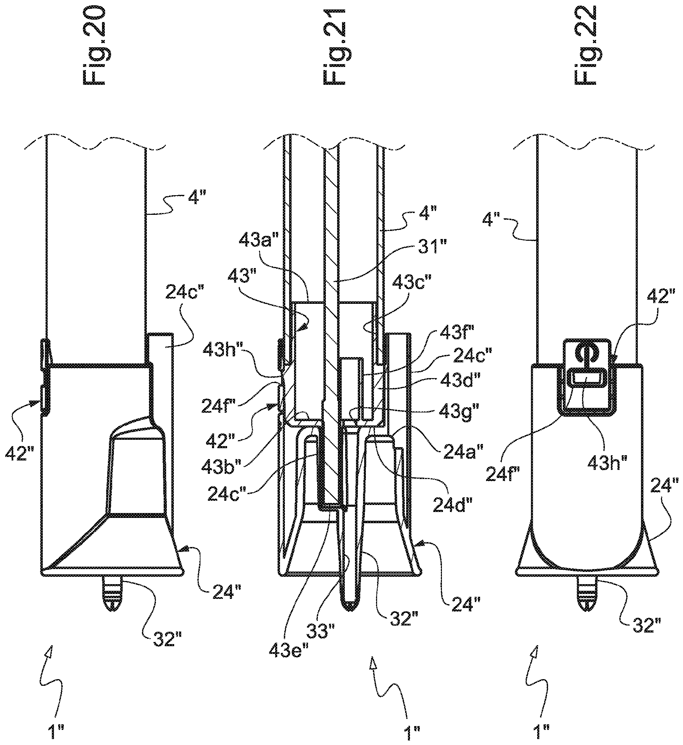

Si l'on se réfère enfin aux Figures 20 à 22, on peut voir que l'on y a représenté la région distale d'un dispositif 1" selon un troisième mode de réalisation, qui présente, vis-à-vis des autres modes de réalisation, la particularité d'avoir une cupule 24" reliée au corps tubulaire 4" de façon amovible par des moyens de fixation par emboitage élastique réversible 42".

Comme dans les modes de réalisation ci-dessus, la cupule 24" comporte une saillie 32" ayant un trou traversant 33" pour permettre le transfert de matériel ou substance, ou le prélèvement, ainsi qu'un orifice 24a" qui est configuré pour relié à une gaine d'aspiration (non représentée) venant se raccorder à une partie extérieure formant raccord 24c" que présente la cupule 24", sur le même principe que la gaine d'aspiration 21' du second mode de réalisation.

La cupule 24" se distingue en ce qu'elle présente, côté proximal, un renfoncement cylindrique 24d" configuré pour permettre à la cupule 24" d'être montée sur une pièce de fixation 43".

La pièce de fixation 43" se présente sous la forme d'une pièce cylindrique creuse ouverte à une extrémité proximale 43a" et dont l'extrémité distale est formée par un fond 43b". La pièce de fixation 43" a une région proximale 43c" ayant un premier diamètre extérieur dimensionné pour lui permettre d'être reçue dans le corps tubulaire 4", par exemple avec montage à force, tandis que la région distale 43d" de la pièce de fixation 43" a un second diamètre supérieur au premier diamètre et dimensionné pour être reçu dans le renfoncement cylindrique 24d" de la cupule 24". La pièce de fixation 43" présente ainsi un épaulement entre les régions proximale 43c" et distale 43d", lequel épaulement viendra en butée contre l'extrémité distale du corps tubulaire 4". En d'autres termes, la pièce de fixation 43" est interposée entre le corps tubulaire 4" et la cupule 24".

Une saillie tubulaire creuse 43e" s'étend à partir du fond 43b" de la pièce de fixation 43", suivant une direction longitudinale parallèle à celle de la pièce de fixation 43". La camera 31", ici de type endoscopique, s'étend dans l'espace intérieur de la saillie creuse 43e", jusqu'au fond de celui-ci. La cupule 24" présente quant à elle un second renfoncement cylindrique 24e" s'étendant à partir du fond du renfoncement cylindrique 24d" et dimensionné pour que la saillie creuse 43e" s'y étende lorsque la cupule 24" est montée sur la pièce de fixation 43".

Par ailleurs, une partie tubulaire 43f" s'étend à partir du fond 43b", parallèlement à la direction longitudinale de la pièce de fixation 43" et à côté de l'ouverture d'entrée de la saillie creuse 43e". La partie tubulaire 43f" est ouverte à son extrémité proximale et donne sur un trou traversant 43g" qui est ménagé dans le fond 43b" dans une position telle qu'il est en regard du trou traversant 33" de la saillie 32" lorsque la cupule 24" est montée sur la pièce de fixation 43", de façon à permettre le passage d'un dispositif de transfert de matériel ou substance à but reproductif, thérapeutique ou diagnostique. En d'autres termes, la partie tubulaire 43f" et l'espace intérieur de la pièce de fixation 43" forme un passage traversant en communication avec le passage traversant du corps tubulaire 4".

Selon une variante avantageuse, la cupule 24" et la pièce de fixation 43" sont toutes les deux transparentes ou translucides et, dans le cas d'une caméra non du type endoscopique, une source d'éclairage, telle qu'au moins une diode électroluminescente, pourra être disposée dans l'espace intérieur de la pièce cylindrique 43" de façon à fournir l'éclairage pour la caméra, tout en restant protégée de l'environnement extérieur.

La cupule 24" est reliée à la pièce de fixation 43" d'une manière amovible par emboîtage élastique réversible comme suit. La cupule 24" présente une ouverture 24f", ici rectangulaire, ménagée dans la paroi du renfoncement cylindrique 24d", et une dent 43h" est formée par un léger renflement sur la surface extérieure de la région distale 43d" de la pièce de fixation 43". Comme on peut le voir sur les Figures 21 et 22, la dent 43f" est formée par une face rectangulaire légèrement inclinée suivant une direction parallèle à la direction longitudinale de la pièce de fixation 43", en s'écartant de la surface extérieure de la région distale 43d" à mesure que l'on s'approche de la région proximale 43c", pour s'achever en une face verticale reliant ladite face inclinée à la surface extérieure de la région distale 43d". L'ouverture 24f" et la dent 43h" sont positionnées de telle sorte que la dent 43h" s'étend dans l'ouverture 24f" lorsque la cupule 24" est dans la position d'utilisation (montée sur la pièce de fixation 43"), avec un bord de l'ouverture 24f" en butée contre la face verticale de la dent 43h".

On comprendra aisément que lorsque l'opérateur monte la cupule 24" sur la pièce de fixation 43", cette dernière sera légèrement déformée vers l'intérieur, dans sa région présentant la dent 43h", jusqu'à ce que le fond du renfoncement 24d" de la cupule 24" vienne en contact avec le fond 43b" de la pièce de fixation 43", moment où la dent 43h" reviendra de manière élastique à sa position non déformée et ainsi viendra en butée contre un bord de l'ouverture 24f", empêchant la cupule 24" d'être déplacée en translation dans la direction proximale-distale. La cupule 24" est ainsi solidement montée en position pour l'utilisation.

Après que les opérations associées au transfert de matériel ou substance ou à un prélèvement sont terminées, et que le dispositif 1" a été retiré de l'animal, l'opérateur peut démonter la cupule 24" d'une seule main en appuyant simplement sur la dent 43h" pour qu'elle s'efface de l'ouverture 24f", tout en tirant sur la cupule 24" pour la séparer de la pièce de fixation 43".

Bien entendu, la pièce de fixation 43" pourra être réalisée dans une matière autorisant une telle déformation.

Enlever une cupule 24" qui vient d'être utilisée et la remplacer par une nouvelle cupule 24" est donc particulièrement rapide et simple. En d'autres termes, les cupules 24" deviennent donc des consommables.

A l'évidence, d'autres moyens de fixation amovible entre la cupule et la pièce de fixation pourront être prévus.

De plus, le principe d'une pièce de fixation qui d'un côté est rendue solidaire de l'ensemble corps et de l'autre côté est reliée de manière amovible à la cupule peut être appliqué au premier mode de réalisation dans lequel une pièce de jonction est prévue pour permettre l'inclinaison de la cupule. Dans un tel cas, la région proximale de la pièce de fixation pourra être formée de manière analogue à la partie corps 23 de la cupule 24 (Figures 4 et 5), les moyens de liaison entre la pièce de fixation et la cupule restant inchangés.

Il est bien entendu que les modes de réalisation ci-dessus de la présente invention ont été donnés à titre indicatif et non limitatif et que des modifications pourront y être apportées sans que l'on s'écarte pour autant du cadre de la présente invention tel que défini par les revendications suivantes.

## Revendications

1. - Dispositif (1, 1', 1") pour accéder à l'utérus d'un animal de rente femelle, en particulier un bovin, par voie vaginale, en vue d'un transfert de matériel ou substance à but reproductif, thérapeutique ou diagnostique, ou d'un prélèvement depuis l'utérus, et pour réaliser ledit transfert ou prélèvement, le dispositif (1, 1', 1") comprenant un ensemble corps (4, 4', 4"), solidaire de moyens de préhension (2, 2') par un opérateur à son extrémité proximale et ayant une extrémité distale (3, 3'), un premier passage (6c, 6c', 4, 4', 4", 25d) traversant le dispositif et débouchant aux extrémités proximale et distale, de façon à établir une voie d'accès entre l'intérieur et l'extérieur de l'animal, pour permettre le transfert de matériel ou substance à but reproductif, thérapeutique ou diagnostique, ou le prélèvement,
l'ensemble corps (4, 4', 4") est sensiblement droit et rigide pour permettre son introduction dans le vagin de l'animal et qu'est prévue à l'extrémité distale (3, 3') une cupule (24, 24', 24") dont l'ouverture est orientée à l'opposé de l'extrémité proximale de l'ensemble corps (4, 4', 4"), une saillie centrale (32, 32', 32") s'étendant à partir du fond de la cupule (24, 24', 24") et ayant un trou traversant (33, 33', 33") dont une extrémité est en communication avec le premier passage traversant (6c, 6c', 4, 4', 4", 25d) de l'ensemble corps, la cupule (24, 24', 24") présentant en outre un orifice (24a, 24a') en communication avec une extrémité distale d'un second passage traversant (21, 21') prévu dans l'ensemble corps (4, 4', 4") et dont l'autre extrémité, proximale, est configurée pour être reliée à des moyens d'aspiration (7, 14, 17) configurés pour créer dans la cupule (24, 24', 24") une dépression permettant de plaquer la cupule (24, 24', 24") contre le fornix de l'animal, le premier passage (6c, 6c', 4, 4', 4", 25d) formant alors une voie d'accès à l'utérus depuis l'extérieur de l'animal, le dispositif (1, 1', 1") étant **caractérisé par le fait que** la cupule (24, 24', 24") comprend également un second trou (24b, 24b', 24e") apte à recevoir des moyens de vision ayant un champ de vision orienté dans la direction distale du dispositif (1, 1', 1") pour permettre à un opérateur de visualiser l'environnement devant la cupule (24, 24', 24").

2. - Dispositif (1, 1', 1") selon la revendication 1, **caractérisé par le fait que** les moyens de vision comprennent une caméra (31, 31', 31") ayant un objectif reçu dans le second trou (24b, 24b', 24e") , une source d'éclairage (25a', 25b') et des moyens de transmission des images capturées par la caméra (31, 31', 31") à un dispositif de visualisation (D) ayant un écran permettant à l'opérateur d'observer lesdites images capturées.

3. - Dispositif (1, 1") selon la revendication 2, **caractérisé par le fait que** la caméra (31, 31") est une caméra endoscopique (31, 31") intégrant la source d'éclairage, lesdits moyens de transmission étant constitués par le corps de la caméra endoscopique (31, 31"), lequel corps s'étend le long de l'ensemble corps (4, 4") jusqu'à la région de l'extrémité proximale de ce dernier, où la caméra endoscopique (31, 31") est reliée au dispositif de visualisation (D).

4. - Dispositif (1') selon la revendication 2, **caractérisé par le fait que** lesdits moyens de transmission consistent en au moins un circuit électronique (13b', 13c', 13d') reçu dans l'ensemble corps (4') et relié à la caméra (31'), l'au moins un circuit électronique (13b', 13c', 13d') étant configuré pour transmettre de manière sans fil les images capturées par la caméra (31') au dispositif de visualisation (D).

5. - Dispositif (1') selon la revendication 4, **caractérisé par le fait que** la cupule (24') est transparente et comprend, sur sa partie arrière, des diodes électroluminescentes (25a', 25b') disposées de façon à éclairer dans la direction distale du dispositif (1'), le faisceau lumineux des diodes (25a', 25b') traversant la cupule (24') transparente et les diodes (25a', 25b') constituant ainsi la source d'éclairage pour la caméra (31').

6. - Dispositif (1, 1', 1") selon l'une des revendications 1 à 5, **caractérisé par le fait que** la cupule (24, 24', 24") est reliée de façon amovible à l'ensemble corps (4, 4', 4").

7. - Dispositif (1") selon la revendication 6, **caractérisé par le fait que** la cupule (24") est reliée de façon amovible à l'ensemble corps (4") par des moyens de type fixation rapide (42"), tels que des moyens de fixation par emboîtage élastique réversible.

8. - Dispositif (1") selon l'une des revendications 6 et 7, **caractérisé par le fait qu'**il comprend une pièce de fixation (43") solidaire de l'ensemble corps (4") et comprenant une région distale (43d") qui s'étend à partir de l'ensemble corps (4"), la cupule (24") présentant, côté proximal, un renfoncement (24d") configuré pour recevoir ladite région distale (43d"), l'une ou l'autre, ou les deux, parmi la cupule (24") et ladite région distale (43d") présentant les moyens de type fixation rapide (42"), la pièce de fixation (43") présentant en outre un passage traversant en communication avec le premier passage traversant de l'ensemble corps (4") et avec le trou traversant (33") de la saillie (32") de la cupule (24") lorsque cette dernière est fixée à la pièce de fixation (43") .

9. - Dispositif (1") selon la revendication 8, prise en dépendance de la revendication 2, **caractérisé par le fait que** lesdits moyens de transmission consistent en au moins un circuit électronique reçu dans l'ensemble corps (4") et relié à la caméra, l'au moins un circuit électronique étant configuré pour transmettre de manière sans fil les images capturées par la caméra au dispositif de visualisation, la pièce de fixation (43") et la cupule (24") étant transparentes, une source d'éclairage, telle que des diodes électroluminescentes, étant montée à l'intérieur de la pièce de fixation (43") de façon à éclairer dans la direction distale du dispositif (1").

10. - Dispositif (1, 1', 1") selon l'une des revendications 1 à 8, **caractérisé par le fait que** l'ensemble corps (4, 4', 4") est de forme cylindrique et comprend une partie corps (4, 4', 4") solidaire des moyens de préhension (2, 2'), la partie corps (4, 4', 4") étant, de préférence, formée en matière plastique telle que du polychlorure de vinyle.

11. - Dispositif (1, 1', 1") selon l'une des revendications 1 à 9, **caractérisé par le fait que** l'orifice (33, 33', 33") de la saillie (32, 32', 32") de la cupule (24, 24', 24") est fermé par un capuchon (34, 34') en matière élastomère solidaire de la saillie (32, 32', 32"), le capuchon (34, 34') protégeant un organe de transfert ou de prélèvement (38) par rapport à d'éventuelles sécrétions de l'animal, le capuchon (34, 34') ayant une fente centrale en croix permettant à un organe de transfert ou de prélèvement (38) de passer à travers celui-ci lorsque ledit organe (38) est poussé par un opérateur, et donc de dépasser de la saillie (32, 32', 32").

12. - Dispositif (1, 1') selon l'une des revendications 1 à 10, **caractérisé par le fait que** les moyens de préhension (2, 2') comprennent une poignée (7, 7') et un corps (5, 5'), les moyens de préhension (2, 2') combinés à l'ensemble corps (4, 4', 4") ayant une forme de pistolet.

13. - Dispositif (1) selon la revendication 12, **caractérisé par le fait que** les moyens d'aspiration comprennent une pompe manuelle ayant un piston (14) et une chambre (17), une partie de la poignée (7) des moyens de préhension (2) étant montée pivotante par rapport au corps (5) des moyens de préhension (2) et étant reliée au piston (14), ce par quoi un déplacement de la poignée (7) actionne le piston (14) et crée une aspiration dans la chambre (17).

14. - Dispositif (1) selon l'une des revendications 1 à 13, **caractérisé par le fait que** l'ensemble corps (4) est de forme cylindrique, et comprend une partie corps (4) solidaire des moyens de préhension (2) et une partie tête (3) formant l'extrémité distale dudit ensemble corps (4), la partie tête (3) étant reliée à la partie corps (4) par une liaison pivot (26) le long d'un diamètre de l'ensemble corps (4), le dispositif (1) comprenant en outre des moyens d'actionnement (27, 28, 29, 30) permettant d'orienter la partie de tête (3) entre une position dite droite dans laquelle la partie corps (4) et la partie tête (3) sont alignées sur une même droite, et une position dite inclinée dans laquelle la partie corps (4) et la partie de tête (3) forment entre elles un angle non nul, de préférence compris entre 0° et 35°.

15. - Dispositif (1) selon la revendication 14, **caractérisé par le fait que** les moyens d'actionnement (27, 28, 29, 30) permettant d'orienter la partie de tête (3) comprennent un étrier (30), un câble (28) et un ressort (27), l'étrier (30) étant monté pivotant sur et par rapport aux moyens de préhension (2), le câble (28) reliant l'étrier (30) et un premier point de liaison (29) formé dans la partie tête (3), et le ressort (27) reliant la partie tête (3) et un second point formé dans la partie corps (4), les premier et second points étant situés de part et d'autre d'un plan imaginaire comprenant la liaison pivot (26) et coupant la partie corps (4) dans le sens de la longueur en deux parties égales, de telle sorte que la partie tête (3) est placée en position inclinée lors d'un pivotement de l'étrier (30) vers l'extrémité proximale du dispositif (1), par traction sur le câble (28), et revient en position droite sous l'action du ressort (27) lorsque l'étrier (30) est relâché.

16. - Dispositif (1') selon l'une des revendications 1 à 15, **caractérisé par le fait que** la cupule (24') comprend un trou traversant d'aspiration de glaires (39'), dont une extrémité distale débouche côté ouverture de la cupule (24') et dont l'extrémité proximale est configurée pour être reliée à une extrémité distale d'une gaine d'aspiration (40') dont l'extrémité proximale est configurée pour être reliée à des moyens d'aspiration de glaires (41') eux-mêmes configurés pour créer une dépression permettant d'aspirer les éventuelles glaires depuis l'ouverture de la cupule (24') vers l'extérieur du dispositif (1') en passant par le trou traversant d'aspiration de glaires (39') et la gaine d'aspiration (40').

## Patentansprüche

1. - Vorrichtung (1, 1', 1") für den Zugang zum Uterus eines weiblichen Nutztieres, vor allem eines Rindes, auf vaginalem Weg zwecks eines Transfers eines Materials oder einer Substanz mit reproduktivem, therapeutischem oder diagnostischem Ziel oder einer Entnahme aus dem Uterus und um den Transfer oder die Entnahme durchzuführen, wobei die Vorrichtung (1, 1', 1") eine Körpereinheit (4, 4', 4") umfasst, die mit Mitteln zum Ergreifen (2, 2') durch einen Bediener an ihrem proximalen Ende fest verbunden ist und ein distales Ende (3, 3') hat, wobei ein erster Durchgang (6c, 6c', 4, 4', 4", 25d) die Vorrichtung durchquert und am proximalen und distalen Ende ausmündet, so dass ein Zugangsweg zwischen dem Inneren und dem Äußeren des Tieres geschaffen wird, um den Transfer eines Materials oder einer Substanz mit reproduktivem, therapeutischem oder diagnostischem Ziel oder die Entnahme zu gestatten,
wobei die Körpereinheit (4, 4', 4") etwa gerade und fest ist, um ihr Einführen in die Vagina des Tieres zu gestatten und am distalen Ende (3, 3') ein Napf (24, 24', 24") vorgesehen ist, dessen Öffnung entgegengesetzt zum proximalen Ende der Körpereinheit (4, 4', 4") ausgerichtet ist, wobei sich ein zentraler Vorsprung (32, 32', 32") ausgehend vom Boden des Napfs (24, 24', 24") erstreckt und ein Durchgangsloch (33, 33', 33") hat, von dem ein Ende mit dem erster durchgängigen Durchgang (6c, 6c', 4, 4', 4", 25d) der Körpereinheit in Kommunikation ist, wobei der Napf (24, 24', 24") ferner eine Öffnung (24a, 24a') in Kommunikation mit einem distalen Ende eines zweiten durchgängigen Durchgangs (21, 21') aufweist, der in der Körpereinheit (4, 4', 4") vorgesehen ist und dessen anderes Ende, das proximale, ausgelegt ist, um mit Ansaugmitteln (7, 14, 17) verbunden zu sein, die ausgelegt sind, um in dem Napf (24, 24', 24") einen Unterdruck zu erzeugen, der gestattet, den Napf (24, 24', 24") an das Scheidengewölbe des Tieres anzudrücken, wobei der erste Durchgang (6c, 6c', 4, 4', 4", 25d) somit einen Zugangsweg zum Uterus von außerhalb des Tieres bildet, wobei die Vorrichtung (1, 1', 1") **dadurch gekennzeichnet ist, dass** der Napf (24, 24', 24") ebenfalls ein zweites Loch (24b, 24b', 24e") umfasst, das imstande ist, Sichtmitte mit einem Sichtfeld aufzunehmen, das in die distale Richtung der Vorrichtung (1, 1', 1") gerichtet ist, um es einem Bediener zu gestatten, die Umgebung vor dem Napf (24, 24', 24") zu visualisieren.

2. - Vorrichtung (1, 1', 1") nach Anspruch 1, **dadurch gekennzeichnet, dass** die Sichtmittel eine Kamera (31, 31', 31") umfassen, die ein Objektiv hat, das in dem zweiten Loch (24b, 24b', 24e") aufgenommen ist, eine Lichtquelle (25a', 25b') und Übertragungsmittel der von der Kamera (31, 31', 31") aufgenommenen Bilder an eine Visualisierungsvorrichtung (D), die einen Bildschirm hat, der es dem Bediener gestattet, die aufgenommenen Bilder zu betrachten.

3. - Vorrichtung (1, 1") nach Anspruch 2, **dadurch gekennzeichnet, dass** die Kamera (31, 31") eine endoskopische Kamera (31, 31") ist, die die Lichtquelle integriert, wobei die Übertragungsmittel von dem Körper der endoskopischen Kamera (31, 31") gebildet sind, wobei sich der Körper entlang der Körpereinheit (4, 4") bis zu der Region des proximalen Endes derselben erstreckt, wo die endoskopische Kamera (31, 31") mit der Visualisierungsvorrichtung (D) verbunden ist.

4. - Vorrichtung (1') nach Anspruch 2, **dadurch gekennzeichnet, dass** die Übertragungsmittel aus mindestens einem elektronischen Schaltkreis (13b', 13c', 13d') bestehen, der in der Körpereinheit (4') aufgenommen und mit der Kamera (31') verbunden ist, wobei der mindestens eine elektronische Schaltkreis (13b', 13c', 13d') ausgelegt ist, um die von der Kamera (31') aufgenommenen Bilder drahtlos an die Visualisierungsvorrichtung (D) zu übertragen.

5. - Vorrichtung (1') nach Anspruch 4, **dadurch gekennzeichnet, dass** der Napf (24') transparent ist und auf seinem hinteren Teil elektrolumineszierende Dioden (25a', 25b') umfasst, die derart angeordnet sind, dass sie in die distale Richtung der Vorrichtung (1') leuchten, wobei der Lichtstrahl der Dioden (25a', 25b') den transparenten Napf (24') durchquert und die Dioden (25a', 25b') somit die Lichtquelle für die Kamera (31') darstellen.

6. - Vorrichtung (1, 1', 1") nach einem der Ansprüche 1 bis 5, **dadurch gekennzeichnet, dass** der Napf (24, 24', 24") mit der Körpereinheit (4, 4', 4") lösbar verbunden ist.

7. - Vorrichtung (1") nach Anspruch 6, **dadurch gekennzeichnet, dass** der Napf (24") mit der Körpereinheit (4") durch Mittel vom Typ Schnellbefestigung (42") wie Befestigungsmittel durch reversibles elastisches Rasten lösbar verbunden ist.

8. - Vorrichtung (1") nach einem der Ansprüche 6 und 7, **dadurch gekennzeichnet, dass** sie ein Befestigungsteil (43") umfasst, das mit der Körpereinheit (4") fest verbunden ist und eine distale Region (43d") umfasst, die sich ausgehend von der Körpereinheit (4") erstreckt, wobei der Napf (24") proximalseitig eine Vertiefung (24d") aufweist, die ausgelegt ist, um die distale Region (43d") aufzunehmen, wobei entweder der Napf (24") oder die distale Region (43d") oder beide die Mittel vom Typ Schnellbefestigung (42") aufweisen, wobei das Befestigungsteil (43") ferner einen durchgängigen Durchgang in Kommunikation mit dem erster durchgängigen Durchgang der Körpereinheit (4") und mit dem Durchgangsloch (33") des Vorsprungs (32") des Napfs (24") aufweist, wenn dieser zuletzt genannte am Befestigungsteil (43") befestigt ist.

9. - Vorrichtung (1") nach Anspruch 8, herangezogen in Abhängigkeit von Anspruch 2, **dadurch gekennzeichnet, dass** die Übertragungsmittel aus mindestens einem elektronischen Schaltkreis bestehen, der in der Körpereinheit (4") aufgenommen und mit der Kamera verbunden ist, wobei der mindestens eine elektronische Schaltkreis ausgelegt ist, um die von der Kamera aufgenommenen Bilder drahtlos an die Visualisierungsvorrichtung zu übertragen, wobei das Befestigungsteil (43") und der Napf (24") transparent sind, wobei eine Lichtquelle wie elektrolumineszierende Dioden im Inneren des Befestigungsteils (43") derart angebracht ist, dass in die distale Richtung der Vorrichtung (1") geleuchtet wird.

10. - Vorrichtung (1, 1', 1") nach einem der Ansprüche 1 bis 8, **dadurch gekennzeichnet, dass** die Körpereinheit (4, 4', 4") zylindrisch geformt ist und einen Körperteil (4, 4', 4") umfasst, der mit Greifmitteln (2, 2') fest verbunden ist, wobei der Körperteil (4, 4', 4") vorzugsweise aus Kunststoffmaterial wie Polyvinylchlorid gebildet ist.

11. - Vorrichtung (1, 1', 1") nach einem der Ansprüche 1 bis 9, **dadurch gekennzeichnet, dass** die Öffnung (33, 33', 33") des Vorsprungs (32, 32', 32") des Napfs (24, 24', 24") von einer Kappe (34, 34') aus Elastomermaterial verschlossen ist, die mit dem Vorsprung (32, 32', 32") fest verbunden ist, wobei die Kappe (34, 34') ein Transfer- oder Entnahmeorgan (38) in Bezug auf eventuelle Sekrete des Tieres schützt, wobei die Kappe (34, 34') einen zentralen kreuzförmigen Schlitz hat, der es einem Transfer- oder Entnahmeorgan (38) gestattet, durch diese zu gelangen, wenn das Organ (38) von einem Bediener geschoben wird und damit über den Vorsprung (32, 32', 32") hinauszuragen.

12. - Vorrichtung (1, 1') nach einem der Ansprüche 1 bis 10, **dadurch gekennzeichnet, dass** die Greifmittel (2, 2') einen Griff (7, 7') und einen Körper (5, 5') umfassen, wobei die Greifmittel (2, 2'), mit der Körpereinheit (4, 4', 4") kombiniert, eine Pistolenform haben.

13. - Vorrichtung (1) nach Anspruch 12, **dadurch gekennzeichnet, dass** die Ansaugmittel eine manuelle Pumpe umfassen, die einen Kolben (14) und eine Kammer (17) hat, wobei ein Teil des Griffs (7) der Greifmittel (2) in Bezug auf den Körper (5) der Greifmittel (2) schwenkend angebracht ist und mit dem Kolben (14) verbunden ist, weswegen eine Verlagerung des Griffs (7) den Kolben (14) betätigt und eine Ansaugung in die Kammer (17) erzeugt.

14. - Vorrichtung (1) nach einem der Ansprüche 1 bis 13, **dadurch gekennzeichnet, dass** die Körpereinheit (4) zylindrisch geformt ist und einen Körperteil (4) umfasst, der mit den Greifmitteln (2) fest verbunden ist und einen Kopfteil (3), der das distale Ende der Körpereinheit (4) bildet, wobei der Kopfteil (3) mit dem Körperteil (4) durch eine Drehzapfenverbindung (26) entlang eines Durchmessers der Körpereinheit (4) verbunden ist, wobei die Vorrichtung (1) ferner Betätigungsmittel (27, 28, 29, 30) umfasst, die erlauben, den Kopfteil (3) zwischen einer geraden Position, in welcher der Körperteil (4) und der Kopfteil (3) auf derselben Geraden ausgerichtet sind, und einer geneigten Position, in welcher der Körperteil (4) und der Kopfteil (3) zwischen sich eine Winkel von nicht null bilden, der vorzugsweise zwischen 0° und 35° liegt, auszurichten.

15. - Vorrichtung (1) nach Anspruch 14, **dadurch gekennzeichnet, dass** die Betätigungsmittel (27, 28, 29, 30), die erlauben, den Kopfteil (3) auszurichten, einen Bügel (30), ein Kabel (28) und eine Feder (27) umfassen, wobei der Bügel (30) auf den Greifmitteln und in Bezug auf die Greifmittel (2) schwenkend angebracht ist, wobei das Kabel (28) den Bügel (30) und einen ersten Verbindungspunkt (29) verbindet, der im Kopfteil (3) gebildet ist, und die Feder (27) den Kopfteil (3) und einen zweiten Punkt verbindet, der im Körperteil (4) gebildet ist, wobei sich der erste und zweite Punkt beiderseits einer imaginären Ebene befinden, die die Drehzapfenverbindung (26) umfasst und den Körperteil (4) in Längsrichtung in zwei gleiche Teile schneidet, so dass der Kopfteil (3) bei einem Schwenken des Bügels (30) zum proximalen Ende der Vorrichtung (1) durch Ziehen an dem Kabel (28) in geneigter Position platziert ist und unter der Wirkung der Feder (27) in die gerade Position zurückkehrt, wenn der Bügel (30) losgelassen wird.

16. - Vorrichtung (1') nach einem der Ansprüche 1 bis 15, **dadurch gekennzeichnet, dass** der Napf (24') ein Durchgangsloch zum Ansaugen von Schleim (39') umfasst, von dem ein distales Ende auf der Öffnungsseite des Napfs (24') ausmündet und dessen proximales Ende ausgelegt ist, um mit einem distalen Ende eines Ansaugschlauchs (40') verbunden zu sein, dessen proximales Ende ausgelegt ist, um mit Schleimansaugmitteln (41') verbunden zu sein, die selbst ausgelegt sind, um einen Unterdruck zu erzeugen, der erlaubt, die eventuellen Schleime ausgehend von der Öffnung des Napfs (24') nach außerhalb der Vorrichtung (1') durch das Durchgangsloch zum Ansaugen von Schleim (39') und den Ansaugschlauch (40') hindurch anzusaugen.

## Claims

1. - A device (1, 1', 1") for accessing, via the vagina, the uterus of a female livestock animal, such as a bovine, for the purpose of a transfer of material or substance with a reproductive, therapeutic or diagnostic purpose, or for the purpose of a sampling from the uterus, and for carrying out said transfer or sampling, the device (1, 1', 1") comprising a body assembly (4, 4', 4"), which is integral, at the proximal end thereof, with grasping means (2, 2') for an operator and has a distal end (3, 3'), a first passage (6c, 6c', 4, 4', 4", 25d) extending through the device and opening out at the proximal and distal ends, so as to establish an access route between the inside and the outside of the animal, so as to allow the transfer of material or substance with a reproductive, therapeutic or diagnostic purpose, or the sampling,
wherein the body assembly (4, 4', 4") is substantially straight and rigid so as to allow it to be inserted into the vagina of the animal, and is provided, at the distal end (3, 3'), a cup (24, 24', 24"), the opening of which is oriented away from the proximal end of the body assembly (4, 4', 4"), a central projection (32, 32', 32") extending from the bottom of the cup (24, 24', 24") and having a through hole (33, 33', 33"), an end of which is in communication with the first through passage (6c, 6c', 4, 4', 4", 25d) of the body assembly, the cup (24, 24', 24") also having an orifice (24a, 24a') in communication with a distal end of a second through passage (21, 21') provided in the body assembly (4, 4', 4") and the other, proximal, end of which being configured for connection to suction means (7, 14, 17) which are configured to create in the cup (24, 24', 24") a negative pressure that presses the cup (24, 24', 24") against the fornix of the animal, the first passage (6c, 6c', 4, 4', 4", 25d) then constituting an access route to the uterus from the outside of the animal, the device (1, 1', 1") being **characterised in that** the cup (24, 24', 24") also comprising a second hole (24b, 24b', 24e") adapted to receive viewing means having a field of view oriented in the distal direction of the device (1, 1', 1") to allow an operator to view the surroundings ahead of the cup (24, 24', 24").

2. - The device (1, 1', 1") according to claim 1, **characterised in that** the viewing means comprise a camera (31, 31', 31") having an objective received in the second hole (24b, 24b', 24e"), a lighting source (25a', 25b') and transmission means for transmitting images captured by the camera (31, 31', 31") to a viewing device (D) having a screen allowing the operator to observe said captured images.

3. - The device (1, 1") according to claim 2, **characterised in that** the camera (31, 31") is an endoscopic camera (31, 31") in which the lighting source is integrated, said transmission means being formed by the body of the endoscopic camera (31, 31"), said body extending along the body assembly (4, 4") to the proximal end region of the latter, where the endoscopic camera (31, 31") is connected to the viewing device (D).

4. - The device (1') according to claim 2, **characterised in that** the transmission means consist in at least one electronic circuit (13b', 13c', 13d') housed in the body assembly (4') and connected to the camera (31'), the at least one electronic circuit (13b', 13c', 13d') being configured to wirelessly transmit to the viewing device (D) the images captured by the camera (31').

5. - The device (1') according to claim 4, **characterised in that** the cup (24') is transparent and comprises, on the rear part thereof, light-emitting diodes (25a', 25b') arranged so as to illuminate in the distal direction of the device (1'), the light beam of the diodes (25a', 25b') going through the transparent cup (24') and the diodes (25a', 25b') thus constituting the lighting source for the camera (31').

6. - The device (1, 1', 1") according to one of claims 1 to 5, **characterised in that** the cup (24, 24', 24") is removably connected to the body assembly (4, 4', 4").

7. - The device (1") according to claim 6, **characterised in that** the cup (24") is removably connected to the body assembly (4") by means (42") of the quick-release attachment type, such as reversible snap-fit attachment means.

8. - The device (1") according to one of claims 6 and 7, **characterised in that** it comprises an attachment piece (43"), which is integral with the body assembly (4") and comprises a distal region (43d") extending from the body assembly (4"), the cup (24") having, on the proximal side, a recess (24d") configured to receive said distal region (43d"), either one, or both, of the cup (24") and said distal region (43d") having the means (42") of the quick-releasing attachment type, the attachment piece (43") further having a through passage in communication with the first through passage of the body assembly (4") and with the through hole (33") of the projection (32") of the cup (24") when the latter is attached to the attachment piece (43") .

9. - The device (1") according to claim 8, when taken in dependence of claim 2, **characterised in that** the transmission means consist in at least one electronic circuit housed in the body assembly (4") and connected to the camera, the at least one electronic circuit being configured so as to wirelessly transmit to the viewing device the images captured by the camera, the attachment piece (43") and the cup (24") being transparent, a lighting source, such as light-emitting diodes, being mounted inside the attachment piece (43") so as to illuminate in the distal direction of the device (1").

10. - The device (1, 1', 1") according to one of claims 1 to 8, **characterised in that** the body assembly (4, 4', 4") is of cylindrical shape and comprises a body part (4, 4', 4") which is integral with the grasping means (2, 2'), the body part (4, 4', 4") being preferably made of plastic material such as polyvinyl chloride.

11. - The device (1, 1', 1") according to one of claims 1 to 9, **characterised in that** the orifice (33, 33', 33") of the projection (32, 32', 32") of the cup (24, 24', 24") is closed by a cap (34, 34'), made of elastomeric material, which is integral with the projection (32, 32', 32"), the cap (34, 34') protecting the transfer or sampling member (38) with respect to potential secretions from the animal, the cap (34, 34') having a central cross slit allowing a transfer or sampling member (38) to go through it when said member (38) is pushed by an operator, and thus to go beyond the projection (32, 32', 32").

12. - The device (1, 1') according to one of claims 1 to 10, **characterised in that** the grasping means (2, 2') comprise a handle (7, 7') and a body (5, 5'), the grasping means (2, 2') having, in combination with the body assembly (4, 4', 4"), the shape of a gun.

13. - The device (1) according to claim 12, **characterised in that** the suction means comprise a manual pump having a plunger (14) and a chamber (17), a part of the handle (7) of the grasping means (2) being pivotably mounted with respect to the body (5) of the grasping means (2) and being connected to the plunger (14), whereby a movement of the handle (7) actuates the plunger (14) and creates a suction in the chamber (17).

14. - The device (1) according to one of claims 1 to 13, **characterised in that** the body assembly (4) is of a cylindrical shape, and comprises a body part (4) which is integral with the grasping means (2) and a head part (3) constituting the distal end of the body assembly (4), the head part (3) being connected to the body part (4) by a pivot connection (26) along a diameter of the body assembly (4), the device (1) further comprising actuating means (27, 28, 29, 30) which orient the head part (3) between a so-called straight position in which the body part (4) and the head part (3) are aligned on a same straight line, and a so-called tilted position in which the body part (4) and the head part (3) form a non-zero angle to one another, preferably an angle in the range between 0° and 35°.

15. - The device (1) according to claim 14, **characterised in that** the actuating means (27, 28, 29, 30) orienting the head part (3) comprise a yoke (30), a wire (28) and a spring (27), the yoke (30) being pivotably mounted on and with respect to the grasping means (2), the wire (28) connecting the yoke (30) and a first connection point (29) provided in the head part (3), and the spring (27) connecting the head part (3) and a second point provided in the body part (4), the first and second points being located on either side of an imaginary plane which includes the pivot connection (26) and divides the body part (4) longitudinally into two equal parts, such that the head part (3) is brought to the titled position when the yoke (30) is pivoted toward the proximal end of the device (1), by pulling on the wire (28), and returns to the straight position under the action of the spring (27) when the yoke (30) is released.

16. - The device (1') according to one of claims 1 to 15, **characterised in that** the cup (24') comprises a phlegm suction through hole (39'), a distal end of which opens on the opening side of the cup (24') and a proximal end of which is configured to be connected to the distal end of a suction duct (40'), the proximal end of which is configured to be connected to phlegm suction means (41') which are in turn configured to create a negative pressure for sucking the potential phlegm from the opening of the cup (24') to the outside of the device (1') via the phlegm suction through hole (39') and the suction duct (40').
